# EUROPEAN PATENT APPLICATION

(11) **EP 4 764 499 A1**
(43) Date of publication of application: **24.06.2026**
(21) Application number: 24220503.7
(22) Date of filing: 17.12.2024
(51) Int. Cl.: G01N 33/58, G01N 33/66, G01N 33/68, G01N 33/72

(54) **METHOD OF DETECTION OF GLYCATED PROTEINS IN BIOLOGICAL SAMPLES AND USE THEREOF**

(71) Applicant: Mendelova Univerzita v Brne, 613 00 Brno (CZ); Univerzita Pardubice, 532 10 Pardubice (CZ)
(72) Inventor: Bezdekova, Jaroslava, 68351 Holubice (CZ); Pavelicova, Kristyna, 66407 Pozorice (CZ); Vaculovicova, Marketa, 61500 Brno (CZ); Miller, Andrew David, 61200 Brno (CZ); Shivajirao Bhosale, Dattatry, 53009 Pardubice (CZ); Sedlak, Milos, 53009 Pardubice (CZ)
(74) Representative: Harber IP s.r.o.

(57) **Abstract**

The present invention relates to a method of detection of glycated proteins, particularly gHSA, which comprises the steps of:
i) providing anthracene boronic acid methacrylate (ABAM): 10-[*N-*methyl-*N-*(o-boronobenzyl)aminomethyl]-anthracene-9-methyl methacrylate;
ii) preparing a solution of ABAM in a biocompatible buffer with pH in the range of from 6.5 to 8.5 or in *in vitro* blood plasma sample of a healthy subject;
iii) measuring initial fluorescence intensity of ABAM solution from step ii) at the wavelength of from 410 to 520 nm;
iv) *in vitro* mixing of a known amount of ABAM solution from step ii) with a known amount of biological sample;
v) measuring fluorescence intensity of the mixture from step iv) at the wavelength of from 410 to 520 nm;
vi) determining the fluorescence intensity increases between the initial control fluorescence intensity measured in step iii) and the fluorescence intensity of the mixture of ABAM and biological sample measured in step v);
vii) calculating the amount of glycated proteins in the biological sample.

The present invention further relates to the use of this method in determination of the total concentrations of glycated proteins, particularly gHSA, in plasma samples and diagnostics for diabetes mellitus.

## Description

### Field of Art

The present invention relates to a rapid and simple fluorescence assay for the quantitative determination of glycated proteins, preferably glycated human serum albumin (gHSA) in biological samples, based on a combination of boronic acid affinity and photoinduced electron transfer. This is made possible using a boronic acid/fluorophore conjugate molecule that increases fluorescence emission intensity upon formation of boronate esters by chemical coupling to the sugar moieties of glycated proteins. Anthracene boronic acid methacrylate (ABAM) is one such conjugate molecule to detect the presence of glycated proteins in biological samples *via* increases in fluorescence emission intensity as part of an assay. Said ABAM-based assay overcomes drawbacks of the commercially available fructosamine detection assay. Even though both of the assays discriminate between the level of glycated proteins occurring in normal and diabetic plasma with very similar limits of detection and quantification, the ABAM-based assay is twelve times faster, more than six times less expensive and it requires sample volumes ten times lower than the fructosamine assay. Moreover, in analysis of plasma samples, the ABAM-based assay appears particularly gHSA specific.

### Background Art

Glycosylation and glycation are two main types of carbohydrate modifications of proteins. Both are very important; however, their roles in physiology and pathology are totally different. While glycosylation is an enzymatic reaction catalyzed by glycosyltransferases and is a part of normal protein biosynthesis, glycation is a non-enzymatic reaction that proceeds under hyperglycemia and is often connected with pathological states. Abnormal protein glycation is observed in many disorders like cancer, inflammation, neurological diseases (including Parkinson's disease and Alzheimer's disease), and diabetes. Nevertheless, the detection of glycated proteins in clinic for disease diagnosis is surprisingly limited.

Current methods used to determine the levels of glycated proteins in biological solutions such as plasma include, amongst others, ion exchange and affinity chromatography. Although these methods have successfully been applied in many laboratories, these technologies are also time-consuming and often require significant technical expertise to operate. Moreover, the instruments are difficult to down-size into portable devices for real-time and point-of-care detection. In parallel to these quantitative methods, semi-quantitative methods have been used in clinical laboratories. The most common are enzyme-linked immunosorbent assays (ELISAs) (H. Nakayama, Z. Makita, M. Kato, S. Taneda, H. Yoshida, K. Yanagisawa, S. Nakagawa, Quantitative enzyme-linked immunosorbent assay (ELISA) for non-enzymatically glycated serum protein, J. Immunol. Methods, 99(1) (1987) 95-100; E. Hud, M.P. Cohen, Evaluation and performance characteristics of a novel ELISA using monoclonal antibody to glycated albumin, 185(2) (1989) 157-164), and the fructosamine assay (R.N. Johnson, P.A. Metcalf, J.R. Baker, Fructosamine: a new approach to the estimation of serum glycosylprotein. An index of diabetic control, Clin. Chim. Acta, 127(1) (1983) 87-95). However, each involves special sample and kit transportation plus handling with refrigeration, especially where immunological reagents (e.g. antibodies) are concerned. Additionally, antibody production is also characterized by a long manufacturing period and high production costs. Furthermore, a significant problem in the case of the colorimetric fructosamine test is cross-reactivity with other substances present in biological samples (e.g., vitamin C and bilirubin) (S.C. Blair, G.M. Schier, E. Gan, R.G. Moses, Interference in the fructosamine assay on the Technicon RA 1000 analyser, Ann. Clin. Biochem. 24(6) (1987) 635-636). Owing to these drawbacks, neither chromatographic methods nor immunoassays or fructosamine assay are ideal for real-time monitoring in clinical applications.

Finally, fluorescence-based assays have been described. For example, US 5,877,025 discloses a solution phase assay of glycated proteins, such as glycated haemoglobin and plasma-soluble glycated proteins. This assay makes use of a fluorescent compound, containing a boronic acid group and a fluorescent moiety (fluorescein), which tags glycated proteins selectively. The fluorescent moiety is excited at a wavelength at which it is preferentially quenched by boronic acid-mediated coupling to the glycated protein. Hence, fluorescence quenching gives a measure of the degree of protein glycation. However, this fluorescence quenching method lacks sensitivity and is negatively affected by external conditions such as pH, ionic strength, presence of interferents, etc.

Overall, there is no doubt that the monitoring of glycated protein levels in blood, for example of glycated haemoglobin, can be clinically useful for the diagnosis and monitoring of diabetes as part of precision medicine approaches to disease management. Furthermore, there has been a growing recent interest in detecting levels of other glycated proteins in biological solutions such as plasma given that they might also be effective clinical markers for various disease states.

Hence, it would be very beneficial to develop a rapid, low-cost, more efficient, and easy-to-use assay for detection of glycated proteins in biological samples, especially in plasma, as a way to determine patient status with respect to diabetes and potentially other disease states too. Relevant methods for determination of glycated proteins in plasma are summarized in Table 1.

**Table 1. Comparison of methods for determination of glycated proteins**

| **Method** | **Reaction principle** | **Advantage** | **Disadvantage** |
|---|---|---|---|
| Ion Exchange Chromatography | pK-alteration | Precise (especially for glycated hemoglobin) | Determination predominantly of proteins glycated at valine, not at lysine; difficult use method |
| Affinity Chromatography | Boronate ester formation | Precise | Very difficult use method |
| Fructosamine assay | Redox reaction of glycated proteins in alkaline solutions | Quick, economic and precise | Suffers from unexpected side reactions |
| Immunological determination | Interaction antigen-antibody | Quick and precise | Price and stability of antibodies, possibility of cross-reactivity with non-glycated proteins |

### Disclosure of the Invention

In order to overcome the drawbacks mentioned above, a more sensitive fluorescence-based sensor and assay were developed for use in diagnostics, which is highly selective towards glycated proteins, gives rapid response, and is cheap and simple to operate. The resulting sensor uses boronate affinity for glycated protein sensing, taking advantage of that boronates can form reversible covalent bonds with *cis*-diols including glycated proteins, that can themselves be linked with concomitant changes in fluorescence intensity. The boronate of choice was anthracene boronic acid methacrylate (ABAM) (Scheme 1), that consists of three main parts: (1) a boronic acid-containing moiety that serves for selective binding with *cis*-diols in saccharide ligands; (2) a fluorescent moiety, connected to the boronic acid-containing moiety *via* a tertiary amine functional group that modulates the intensity of anthracene fluorescence by photoinduced electron transfer (PET); (3) a methacrylate moiety that allows the incorporation of ABAM into a polymer framework, if desired.

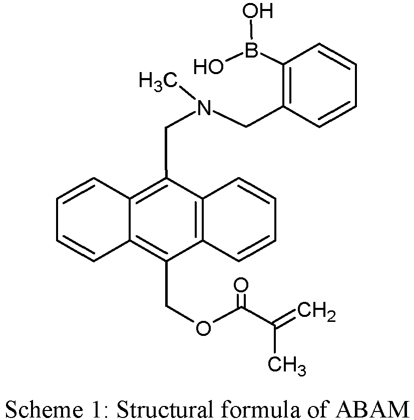

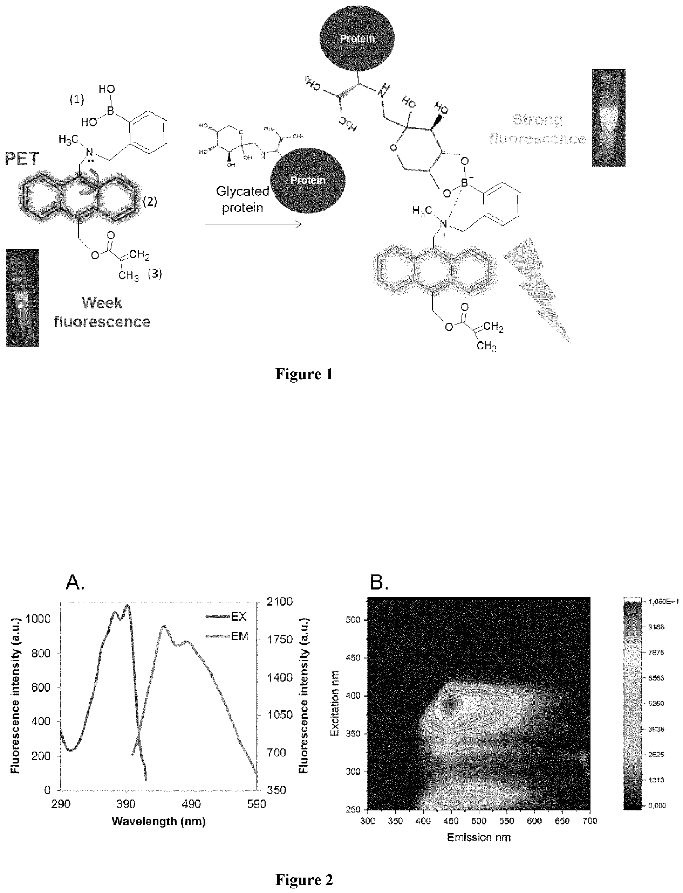

The boronic acid-containing moiety of ABAM acts as a receptor for the binding of *cis*-diols and in so doing generates a boronate complex whose boron atom interacts with the nitrogen atom of the tertiary amine functional group by native bond formation, so preventing the nitrogen lone-pair from acting to quench/suppress anthracene fluorescence (N-B bond theory) (Figure 1). Here, we report a method of detection of glycated protein levels in real biological samples (specifically blood plasma) that uses ABAM and makes use of boronic acid affinity for polyols and PET-suppression, with potential benefits for the facile detection of diabetes in patients at point of care.

Although ABAM molecule has been known since 1999, and thus far has always been used only for detection of fructose. We now describe an ABAM-based assay for detection of glycated proteins. A possible reason why ABAM molecule has not been used for the determination of glycated proteins is that the glycated regions of proteins are typically buried or hidden within protein structures hence potentially preventing bonding interactions between ABAM and saccharide moieties owing to steric hindrance. However, in spite of this, our ABAM-based assay does in fact appear especially suitable for the detection of gHSA in plasma, even in comparison to the fructosamine assay which is the most widely commercial assay system used to date for the detection of glycated proteins. In fact, gHSA which is the most abundant glycated protein in human plasma, is a potentially excellent marker for diabetes because gHSA levels do not fluctuate as a result of comorbidities. The inventors have found that ABAM interacts preferentially with gHSA in preference to all other glycated proteins in plasma. Therefore, ABAM-based assay according to the present invention preferentially and primarily detects gHSA in plasma samples.

### Definitions:

ABAM - 10-[*N*-methyl-*N*-(o-boronobenzyl)aminomethyl]-anthracene-9-methyl methacrylate; anthracene boronic acid methacrylate
CH₂Cl₂ - dichloromethane
DMAP - 4-dimethylaminopyridine
DMSO - dimethyl sulfoxide
DMF - dimethylformamide
MeCN - acetonitrile
MeOH - methanol
(CH₃)₃SI - trimethylsulfonium iodide
PBS - phosphate-buffered saline
TBAF - tetrabutylammonium fluoride
TBDMSCl - *tert*-butyldimethylsilyl chloride
THF - tetrahydrofuran
gHSA - glycated human serum albumin
biocompatible buffer - an aqueous buffer prepared using buffer components compatible with ensuring the structural and functional integrity of biological macromolecules (e.g. proteins) suspended therein
prediabetes - is a bodily state wherein blood sugar levels are higher than usual (concentration of gHSA is in the range of from 70 to 100 µM), but not high enough for diagnosis of type 2 diabetes. The use of the terms implies that a patient is high risk for developing type 2 diabetes. diabetes - is a bodily, lifelong condition that causes a person's blood glucose (sugar) level to become too high (concentration of gHSA is higher than 100 µM) with extensive pathological consequences. There are two main types of diabetes - type 1 diabetes (insulin-dependent) and type 2 (insulin-independent) diabetes. Type 2 diabetes is far more common than type 1.
biological samples - samples of blood, plasma, urine, tissue, cells or saliva, isolated from the body of the subject

The object of the present invention is a method of detection of glycated proteins, such as gHSA, which comprises the following steps:
i) providing ABAM molecule;
ii) preparing a solution of ABAM in a biocompatible buffer with pH in the range of from 6.5 to 8.5 or in *in vitro* blood plasma of a healthy subject; preferably, the solution of ABAM has concentration in the range of from 5 to 50 µM, more preferably from 5 to 25 µM;
iii) measuring initial fluorescence intensity of ABAM solution from step ii) at the wavelength range from 410 to 520 nm, more preferably from 420 to 450 nm, most preferably at 426 nm;
iv) *in vitro* mixing a known amount of ABAM solution from step ii) with a known amount of a biological sample, preferably human plasma sample, in which the amount of glycated proteins, such as gHSA, shall be determined; wherein the final concentration of ABAM is the same as in step ii);
v) measuring fluorescence intensity of the mixture from step iv) at the same wavelength as in step iii);
vi) comparing the fluorescence intensity of the mixture of ABAM and biological sample (e.g. human plasma) measured in step v) with the initial fluorescence intensity of ABAM solution measured in step iii).

From the compared fluorescence intensities, the presence and optionally the amount of glycated proteins present, such as gHSA, may be determined. If there is an increase in the intensity of the mixture of ABAM and biological sample measured in step v) compared to the initial fluorescence intensity of ABAM solution measured in step iii), then potentially pathological levels of glycated proteins are present in the biological sample. A threshold may be set using statistical evaluation of the fluorescence intensity increase (related to the amount of glycated proteins) in biological samples of healthy subjects (to be understood as subjects not suffering of diabetes or prediabetes), statistical evaluation of the fluorescence intensity increase (related to amount of glycated proteins) in biological samples of prediabetic subjects, and statistical evaluation of the fluorescence intensity increase (related to of the amount of glycated proteins) in biological samples of diabetic subjects.

In the case of gHSA detection, a calibration curve may be obtained. Using the calibration curve, if the increase in fluorescence intensity above background is in the range of from 250 to 1700 a.u., then the biological sample corresponds to a patient in a prediabetic state. If the increase in fluorescence intensity above background is higher than 1700 a.u., then the biological sample corresponds to a patient in a diabetic state. These fluorescence intensity increases were determined as a difference between the ABAM fluorescence intensity observed using samples of the blood plasma of a healthy subject, post spiking with known amounts of gHSA, and corresponding samples of the blood plasma of a healthy subject without spiking (blank, 2477 a.u.)

ad i) ABAM molecule can be synthesised according to known procedures (S. Gao, W. Wang, B. Wang, Building fluorescent sensors for carbohydrates using template-directed polymerizations, Bioorg. Chem. 29(5) (2001) 308-320, Y.-I. Lin, S. Lang Jr, C.M. Seifert, R. Child, G. Morton, P. Fabio, Aldehyde syntheses. Study of the preparation of 9, 10-anthracenedicarboxaldehyde, J. Org. Chem. 44(25) (1979) 4701-4703), and it is commercially available as well. ABAM is a boronic acid/fluorophore conjugate molecule that increases fluorescence emission intensity upon formation of boronate esters by chemical coupling to the sugar moieties of glycated proteins.

ad ii) ABAM is typically pre-dissolved in MeOH, to give an aggregation free primary stock solution, typically the concentration of ABAM in the stock solution is in the range of from 2 to 5 g/L (preferred 2.5 g/L) then the stock solution of ABAM in MeOH is diluted for use to from 5 to 50 µM, preferably from 5 to 25 µM solution in a biocompatible buffer or their combinations (see Table 2) in the pH range of from 6.5 to 8.5, preferably in the pH range of from 7 to 8.

**Table 2. Biocompatible buffers for use with ABAM-based assay**

| **Buffer*** | **PBS** | **HEPES** | **Tris** | **MOPS** | **Trizma base** | **PIPES** | **TES** | **BES** |
|---|---|---|---|---|---|---|---|---|
| pH range | 7.4 | 6.8-8.2 | 7-9 | 6.5-7.9 | 7-9 | 6.1-7.5 | 6.8-8.2 | 6.4-7.8 |

| | | | | | | | | |
|---|---|---|---|---|---|---|---|---|
| *abbreviations: HEPES: 4-(2-hydroxyethyl)-1-piperazine ethanesulfonic acid; Tris: tris(hydroxymethyl)-aminomethane; MOPS: 3-(N-morpholino)propane sulfonic acid; Trizma base: 2-amino-2-(hydroxymethyl)-1,3-propanediol; PIPES: piperazine-*N,N*'-bis(2-ethanesulfonic acid); TES: *N-*[Tris(hydroxy-methyl)-methyl]-2-aminoethanesulfonic acid; BES: *N,N-*bis(2-hydroxyethyl)-2-aminoethanesulfonic acid. | | | | | | | | |

The stock solution of ABAM may also be diluted using the blood plasma of a healthy subject. The range for plasma dilution is from 30- to 50-times, preferably 50-times dilution in ultrapure water. The preferred ionic strength of the biocompatible buffer is in the range of from 20 to 150 mM. Suitable biocompatible buffers are HEPES, Tris, MOPS, Trizma base, PIPES, TES and BES.

ad iii) The fluorescence intensity of ABAM solution is measured at the wavelength of from 410 to 520 nm, preferably at wavelength of 420-450 nm, most preferably at 426 nm.

ad iv) biological samples, such as human plasma samples wherein gHSA are to be detected, can be obtained from healthy volunteers or from potential prediabetic and diabetic patients (e.g. from their blood samples). Blood plasma is separated from blood samples by spinning a vessel of fresh blood containing an anticoagulant in a centrifuge until the blood cells fall to the bottom of the tube, and the blood plasma can then be poured or drawn off. The range for plasma dilution is from 30- to 50-times, preferably 50-times dilution in ultrapure water.

ad v) The fluorescence intensity of the mixture from step iv) is measured at the same wavelength as in step iii), thus at the wavelength of from 410 to 520 nm, preferably at wavelength of 420-450 nm, most preferably at 426 nm.

ad vi) The increase in fluorescence intensity of the mixture from step v) compared to the fluorescence intensity of ABAM solution from step iii) is used to determine the concentration of glycated proteins, such as gHSA. ABAM interacts highly preferentially with gHSA in plasma samples, therefore, the increase in fluorescence intensity of the mixture is primarily the result of ABAM-gHSA interactions. Hence, the increase in fluorescence intensity of the mixture can be used to measure gHSA plasma concentrations directly. In a healthy subject, the total concentration of glycated proteins in plasma has been shown to be approx. 160 µM (according to fructosamine assay) of which 44% is gHSA (70 µM) (corresponding to 14% of total HSA in plasma). This gHSA baseline concentration becomes 1.4 µM in 50-fold diluted plasma. So as to demonstrate the applicability of the assay within the prediabetic and diabetic range of gHSA plasma concentrations, a calibration curve was constructed using at least three plasma samples obtained from a healthy subject. In practical terms, these plasma samples were diluted 50-fold (so that the gHSA baseline concentration was reduced to 1.4 µM) then spiked with increasing amounts of gHSA (Sigma-Aldrich, St. Louis, MO, USA) in the concentration range 0.01 to 0.06 g/L, which is equivalent to increasing gHSA concentrations above the healthy baseline concentration in the range 0.15 µM to 1.0 µM. After spiking was concluded, the total gHSA concentration finally reached was 2.4 µM (equivalent to 120 µM in undiluted plasma, which also corresponds to >15% of total HSA in plasma). Fluorescence intensities were measured according to the method as described above (steps i) to vi)). The difference (increase) in fluorescence intensity of the mixture from step v) compared to the fluorescence intensity of ABAM solution from step iii) was then plotted as a function of added gHSA concentrations forming the calibration curve. The resulting calibration curve proves that this method is applicable to distinguish between plasma samples from healthy, prediabetic and diabetic subjects. Moreover, the calibration curve can be used for the determination of an unknown concentration of gHSA in a plasma sample from a given subject by plotting the increase in fluorescence intensity from step vi) on the calibration curve, thereby deriving the unknown gHSA concentration directly. If the unknown gHSA concentration is found in the range from 70 to 100 µM, then the subject is in a prediabetic state. If the unknown gHSA concentration is higher than 100 µM, then the subject is in a diabetic state.

In one embodiment, the ABAM concentration in the final mixture of step iv) is from 5 to 10 µM, preferably 7.5 µM for gHSA determination. At ABAM concentrations <5 µM, ABAM fluorescence enhancements are too small to be detected, while at ABAM concentrations >10 µM, ABAM fluorescence is no longer linearly proportional to analyte concentration.

In one embodiment, fluorescence intensity in step v) is measured after at least 10 mins from mixing ABAM solution with human plasma sample. Preferably, the fluorescence intensity is measured in the time range of from 10 min to 30 min after mixing ABAM solution with the human plasma sample. It was observed that after 10 min the balance of the reaction and fluorescence intensity is well established and the fluorescence does not increase any further.

After 30 min from mixing ABAM and the sample, the fluorescence intensity of ABAM begins to slowly decrease.

In one embodiment, the fluorescence intensity is measured at excitation wavelength in the range of from 370 to 390 nm, and emission wavelength in the range of from 410 nm to 520 nm, more preferably at wavelength of from 420 to 450 nm, most preferably 426 nm.

The object of the present invention is also the use of the above-described ABAM-based method to determine specific concentrations of gHSA in plasma samples.

The object of the present invention is also the use of the above described method in medicine, preferably for *in vitro* diagnostics of prediabetes and diabetes mellitus. If the determined concentration of gHSA in the plasma sample from a subject is in the range from approx. 70 to 100 µM (corresponding to a fluorescence intensity increase above background of from 250 a.u. to 1700 a.u.), then the subject suffers from prediabetes. If the determined concentration of gHSA in the plasma sample of a subject is higher than >100 µM (corresponding to fluorescence intensity increase above background of greater than 1700 a.u.), then the subject suffers from diabetes mellitus. The concentration of gHSA in a given sample can be determined using a statistical evaluation of the fluorescence intensity increase (related to the amount of glycated proteins) in biological samples of healthy subjects (to be understood as subjects not suffering of diabetes or prediabetes), a statistical evaluation of the fluorescence intensity increase (related to amount of glycated proteins) in biological samples of prediabetic subjects, and a statistical evaluation of the fluorescence intensity increase (related to of the amount of glycated proteins) in given biological samples from diabetic subjects.

Preferably, the concentration of gHSA of the sample can be determined by plotting the fluorescence intensity increase of said sample into a calibration curve obtained from the fluorescence intensity increase of samples with various known gHSA concentrations.

The object of the present invention is also a method for diagnosing diabetes from human plasma sample, which comprises the following steps:
i) providing ABAM molecule;
ii) preparing a solution of ABAM in a biocompatible buffer with pH in the range of from 6.5 to 8.5 or in *in vitro* blood plasma sample of a healthy subject; preferably, the solution of ABAM has concentration in the range of from 5 to 50 µM, more preferably from 5 to 25 µM;
iii) measuring initial fluorescence intensity of ABAM solution from step ii) at the wavelength range from 410 to 520 nm, more preferably from 420 to 450 nm, most preferably at 426 nm;
iv) *in vitro* mixing a known amount of ABAM solution from step ii) with a known amount of a human plasma sample, in which the amount of gHSA, shall be determined; preferably, the final concentration of ABAM is the same as in step ii), more preferably the final concentration of ABAM is in the range of from 5 to 50 µM, even more preferably from 5 to 25 µM;
v) measuring fluorescence intensity of the mixture from step iv) at the same wavelength as in step iii);
vi) comparing the fluorescence intensity of the mixture of ABAM and human plasma sample measured in step v) with the initial fluorescence intensity of ABAM solution measured in step iii);
vi) determining the amount of gHSA in the human plasma sample, wherein if the determined concentration of gHSA in the human plasma sample is in the range of from approx. 70 to 100 µM, then the subject suffers from prediabetes, and if the determined concentration of gHSA in the human plasma sample is higher than 100 µM, then the subject suffers from diabetes.

### Summary:

ABAM comprises a boronic acid moiety conjugated to an anthracene fluorophore that surprisingly undergoes fluorescence enhancement interactions preferably with glycated human serum albumin in plasma, even though glycated regions in protein structures are typically buried, which could hinder interactions between ABAM and saccharide structures owing to steric hindrance. Our ABAM-based assay is also unexpectedly superior in many ways to the fructosamine assay, which is the current market leader for the detection of total glycated proteins in blood plasma samples, because it is not influenced or subject to interference in the presence of key analytes, such as non-glycated proteins, multiple saccharides or vitamin C. Our ABAM-based assay is also specific for gHSA detection in plasma samples. The fructosamine assay is not specific for gHSA detection in plasma samples.

### Brief description of Figures

Figure 1: Principle of ABAM-based assay for determination of glycated proteins from real biological samples according to N-B bond theory. λₑₓ, 370 nm; λₑₘ, 426 nm.
Figure 2: Characterization of fluorescent properties of ABAM. **A)** Fluorescence excitation (black line) and emission spectra (grey line) of ABAM in MeOH; **B)** EEM of ABAM.
Figure 3: Increase (%) of ABAM fluorescence intensity with ABAM/D-fructose (D-Fru) complex formation under various conditions. Selection of optimal ABAM concentration (**A**); effect of protic and aprotic solvents on fluorescence intensity increases following ABAM/D-Fru interactions after ABAM stock solutions in MeOH or MeCN were diluted into 30 mM PBS buffer, pH 7.4 (**B**); Effect of pH on fluorescence intensity increases following ABAM/D-Fru interactions in the pH range 6-9 (**C**) and following changes in ionic strength of 10-150 mM (**D**).
Figure 4: A) The Pearson correlation analysis between our ABAM-based assay and the fructosamine assay; B) The fluorescence determination by ABAM -based assay of glycated protein levels in human plasma spiked with different concentrations of gHSA or BSA (negative control).
Figure 5: Electropherograms obtained from CE-LIF analysis of blood and plasma samples using ABAM fluorescence detection. Shown are MEKC electropherograms of Plasma (black line) and Blood (grey line). Glycated proteins are identified: (1) gHSA; (2) HbA1c. A 330-fold dilution of blood and a 100-fold dilution of plasma was employed for the measurements.

### Examples

### Example 1: Synthesis of ABAM

The ABAM molecule was prepared according to Scheme 2.

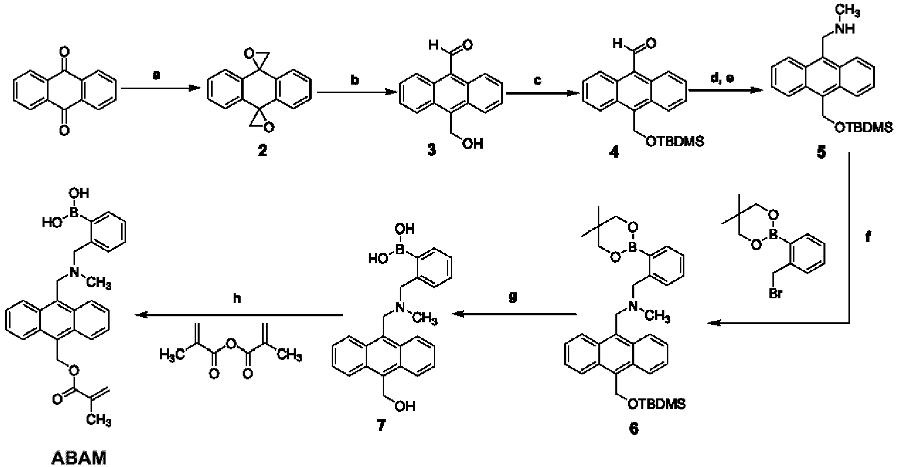

**Scheme 2:** Reagents and conditions: (**a**) NaH, (CH₃)₃SI, DMSO, N₂, dark conditions, rt, 4 h, 85%; (**b**) LiBr, MeCN, N₂, 60 °C, 17 h, 76%; (**c**) TBDMSCl, Imidazole, DMF, N₂, rt, 20 h, 82%; (**d**) MeNH₂ (2 M in MeOH), MeOH, N₂, rt, 24 h; (**e**) NaBH₄, MeOH, rt, 3 h, 86%; (**f**) K₂CO₃, MeCN, N₂, reflux, 25 h, 47%; (**g**) TBAF, THF, N₂, rt, 25 h, 84%; (**h**) DMAP, CH₂Cl₂, N₂, rt, 48 h, 68%.

This seven step synthetic route was devised starting from anthraquinone *via* di-epoxide **2** with reference to previous literature. The preparation of ABAM was reported previously (S. Gao, W. Wang, B. Wang, Building fluorescent sensors for carbohydrates using template-directed polymerizations, Bioorganic Chemistry 29(5) (2001) 308-320, Y.-I. Lin, S. Lang Jr, C.M. Seifert, R. Child, G. Morton, P. Fabio, Aldehyde syntheses. Study of the preparation of 9, 10-anthracenedicarboxaldehyde, The Journal of Organic Chemistry 44(25) (1979) 4701-4703) although starting with hydroxy aldehyde **3.** Accordingly, starting here with anthraquinone, di-epoxide **2** was prepared in 85% yield, after which **3** was prepared by single epoxide ring opening in 76% yield (Y.-I. Lin, S. Lang Jr, C.M. Seifert, R. Child, G. Morton, P. Fabio, Aldehyde syntheses. Study of the preparation of 9, 10-anthracenedicarboxaldehyde, The Journal of Organic Chemistry 44(25) (1979) 4701-4703; N.O. Mahmoodi, S. Mirkhaef, A. Ghavidast, Synthesis of anthracene derivatives of 1, 3-diazabicyclo [3.1. 0] hex-3-ene, Journal of Molecular Structure 1081 (2015) 248-253). Conversion of **3** through to ABAM was then accomplished by following the route of Gao et al. (S. Gao, W. Wang, B. Wang, Building fluorescent sensors for carbohydrates using template-directed polymerizations, Bioorganic Chemistry 29(5) (2001) 308-320), which proved more difficult than expected following formation of silyl ether aldehyde **4.** In particular, conversion of silyl ether amine **5** to amine boronate **6,** by alkylation with 2-(2-bromomethyl-phenyl)-5,5-dimethyl-[1,3,2]-dioxaborinane, proved challenging owing to difficulties in purification of **6** due to apparent compound instability. Hence, **6** was prepared and converted crude to deprotected amine boronate **7** prior to full characterization. The structures of intermediates **2-7** and ABAM were confirmed comprehensively by infrared (IR), ¹H and ¹³C nuclear magnetic resonance (¹H and ¹³C NMR), elemental analysis (where possible) and high-resolution mass spectrometry (HRMS). In the final stage, *p-tert*-butylcatechol (stabilizer) (0.05% w/w) was added to prevent potential self-polymerization of ABAM, which was then stored under an N₂ in the fridge (2-8 °C) until required for further studies.

Unless otherwise stated, reagents and solvents were purchased from commercial sources (Sigma Aldrich, St Louis, MO, USA; Acros Organics, Carlsbad, CA, USA; Merck Chemicals, Darmstadt, Germany; TCI Europe, Zwijndrecht, Belgium). Commercial grade reagents were used without further purification. Reactions were monitored by thin-layer chromatography plates coated with 0.2 mm silica gel 60 F254 (Merck Chemicals, Darmstadt, Germany). TLC plates were visualized by UV irradiation (254 nm). All melting points were determined on a Melting Point B-540 apparatus (Büchi, Flawil, Switzerland) and are uncorrected. IR spectra were recorded on a Nicolet 6700FT-IR spectrometer (Thermo Fisher Scientific, Waltham, MA, USA) over the range of 400-4000 cm⁻¹ using the ATR technique. NMR spectra were measured in CDCl₃, DMSO-d₆ or CD₃OD solution at ambient temperature on a Bruker Avance^{™} III 400 spectrometer at frequencies ¹H (400 MHz) and ¹³C (100.26 MHz) or a Bruker Ascend^{™} 500 spectrometer at frequencies ¹H (500.13 MHz), ¹³C (125.76 MHz) (Bruker, Billerica, MA, USA). The chemical shift, δ, was measured relative to CDCl₃ 7.27 ppm or DMSO-d₆ 2.5 ppm using tetramethylsilane (TMS) as an internal standard. Coupling constants (*J*) were all reported in (Hz). Elemental analysis (C, H, N) was performed on an automatic microanalysis Flash 2000 Organic elemental analyzer (Thermo Fisher Scientific, Waltham, MA, USA). Mass spectrometry with high resolution was performed using the "dried droplet" method with a MALDI mass spectrometer LTQ Orbitrap XL (Thermo Fisher Scientific, Waltham, MA, USA) equipped with a nitrogen laser (337 nm, 60 Hz). Spectra were measured in the positive ion mode in regular mass extent at a resolution of 100,000 at m/z 400. The matrix used was 2,5-dihydrobenzoic acid (DHB).

### Trans-dispiro[oxirane-2,9'(10'H)-anthracene-10',2"-oxirane] 2

A mixture of sodium hydride (NaH) (50% oil dispersion) (1.5 gm, 25.8 mmol) and anthraquinone (3 gm, 10.7 mmol) in dry DMSO (100 mL) was stirred at room temperature in the dark under a N₂ atmosphere, whilst a solution of (CH₃)₃SI (6.7 gm, 25.8 mmol) in dry DMSO (100 mL) was added dropwise over 40 min. The reaction was then further stirred for 3 h. Thereafter, the reaction mixture was filtered and the filtrate poured into ice water (500 mL) then allowed to stand for 30 min. Crystals were collected by filtration and washed with cold water, and dried. Di-epoxide **2** (2.9 gm, 85%) was obtained as light-brown crystals. Mp: 118-119 °C. IR(ATR): 3041, 2921, 1482, 1451, 1321, 917, 898, 844, 765, 748 cm⁻¹. ¹H NMR (CDCl₃, 400 MHz): δ 7.41-736 (8H, m, Ar-*H*), 3.24 (4H, s, *-*O*-CH₂-*C)*.* ¹³C NMR (CDCl₃, 100.26 MHz): 135.5, 128.5, 122.4, 63.9, 54.5. HRMS: m/z calc. for C₁₆H₁₂O₂: 237.09156 [M+H]⁺; found; 237.09129 [M+H]⁺.

### 10-(Hydroxymethyl)-anthracene-9-carbaldehyde 3

Di-epoxide **2** (1 gm, 4.2 mmol) and lithium bromide (LiBr) (1.6 gm, 19.5 mmol) were dissolved together in dry MeCN (60 mL) in dark conditions. Thereafter, the reaction mixture was stirred at 60 °C for 17 h under a N₂ atmosphere, cooled to -40 °C in a dry ice-acetone bath then allowed to stand for a further 30 min. Crystals were collected by sintered funnel filtration, then the resulting filtrate was washed with water and dried. Hydroxy aldehyde **3** (0.760 gm, 76%) was obtained as a yellow solid. Mp: 181-183 °C. IR(ATR): 3390, 3068, 2948, 2926, 2896, 2855, 1671, 1645, 1585, 1389, 1283, 1052, 931, 835, 816, 757 cm⁻¹. ¹H NMR (DMSO-d₆, 500.13 MHz): δ 11.4 (1H, s, Ar*-CHO*), 8.94 (2H, d, *J* = 8.5 Hz, Ar-*H*), 8.55 (2H, d, *J* = 9.0 Hz, Ar-*H*), 7.71 (2H, t, *J* = 7.0 Hz, *J* = 15.5 Hz, Ar-*H*), 7.66 (2H, *t, J =* 7.5 Hz, *J =* 14.5 H_{Z}, Ar-*H*), 5.59 (1H, t, *J* = 5.5 Hz, *J* = 11.0 Hz, Ar-CH₂-*OH*), 5.47 (2H, d, *J* = 5.5 Hz, Ar-*CH₂*-OH). ¹³C NMR (DMSO-d₆, 125.76 MHz): δ 196.5, 141.5, 130.8, 129.2, 128.4, 126.0, 125.8, 125.5, 123.8, 55.5. HRMS: m/z calc. for C₁₆H₁₂O₂: 237.09156 [M+H]⁺; found: 237.09111 [M+H]⁺.

### 10-(tert-Butyldimethyl-silanyloxymethyl)-anthracen-9-carbaldehyde 4

Hydroxy aldehyde **3** (0.7 gm, 2.96 mmol), TBDMSCl (0.580 gm, 3.85 mmol) and imidazole (0.2 gm, 3.85 mmol) were dissolved in anhydrous dimethylformamide (DMF) (30 mL). The reaction mixture was then stirred at room temperature for 20 h under a N₂ atmosphere. After completion (monitored by TLC), organic solvent was removed under reduced pressure, and the residue dissolved in ethyl acetate (100 mL) then washed with cold water (100 mL), 0.5 N HCl solution (20 mL), and lastly with brine solution (30 mL). Following this, the organic layer was dried over anhydrous sodium sulfate and concentrated, then the residue was purified by column chromatography eluting with ethyl acetate:*n*-hexane (1:1, *v*/*v*) to yield silylether aldehyde **4** (0.820 gm, 82%) as a yellow solid product. Mp: 190-120 °C. IR(ATR): 2953, 2927, 2883, 2854, 1667, 1470, 1381, 1257, 1040, 954, 834, 805, 775 cm⁻¹. ¹H NMR (CDCl₃, 400 MHz): δ 11.4 (1H, s, Ar*-CHO*), 8.88 (2H, d, *J* = 9.5 Hz, Ar-*H*), 8.47 (2H, d, *J* = 9.5 Hz, Ar-*H*), 7.76-7.57 (4H, m, Ar-*H*), 5.63 (2H, s, Ar-C*H*₂-O-TBDMS), 0.91 (9H, s, -C*-*(*CH₃)₃*), 0.14 (6H, s, -Si-(*CH₃)₂*)*.* ¹³C NMR (CDCl₃, 100.26 MHz): δ 193.8, 139.7, 131.6, 129.9, 128.3, 126.4, 126.1, 125.4, 124.1, 58.1, 26.0, 18.5, -4.8. HRMS: m/z calc. for C₂₂H₂₆O₂Si: 351.17748 [M+H]⁺; found: 351.17856 [M+H]⁺. Previously reported data FAB-MS m/z: 350.17 [M]⁺.

### [10-(tert-Butyldimethyl-silanyloxymethyl)-anthracen-9-yl-methyl]-methylamine 5

Silylether aldehyde **4** (1 gm, 2.83 mmol) was dissolved in a 2 M solution of methylamine (MeNH₂) in MeOH (45 mL) and then the reaction mixture was stirred at room temperature for 24 h. After completion (monitored by TLC), sodium borohydride (NaBH₄) (0.324 gm, 8.5 mmol) was added after which the whole was stirred at room temperature for a further 3 h. After final completion (monitored by TLC), the organic solvent was removed under reduced pressure, and the residue dissolved in CH₂Cl₂ (150 mL), then washed with water (200 mL). Following this, the organic layer was dried over anhydrous sodium sulfate and concentrated, then the residue was purified by column chromatography eluting with CH₂Cl₂:MeOH (3.5:0.5, v/v) to yield silylether amine **5** (0.9 gm, 86%) as a yellow solid product. Mp: 192-193 °C. IR(ATR): 3069, 2953, 2927, 2885, 2855, 1776, 1591, 1488, 1470, 1387, 1251, 1077, 1045, 938, 834, 810, 775 cm⁻¹. ¹H NMR (DMSO-d₆, 400 MHz): δ 8.52-8.45 (4H, m, Ar-*H*), 7.64-7.61 (4H, m, Ar-*H*), 5.65 (2H, s, Ar-C*H*₂-O-TBDMS), 4.89 (2H, s, Ar-C*H*₂-NH-CH₃), 2.64 (3H, s, -NH-*CH₃*), 0.83 (9H, s, -C-(*CH₃)₃*), 0.11 (6H, s, *-*Si*-(CH₃)₂*)*.* ¹³C NMR (DMSO-d₆, 100.26 MHz): δ 132.89, 130.09, 129.43, 128.83, 125.93, 125.75, 125.19, 124.99, 57.27, 45.45, 35.00, 25.74, 17.94, -5.08. HRMS: m/z calc. for C₂₃H₃₁NOSi: 366.22532 [M+H]⁺; found: 366.22537 [M+H]⁺. Previously reported data³ FAB-MS m/z: 365.3 [M]⁺.

### [10-(tert-Butyldimethyl-silanyloxymethyl)-anthracen-9-yl-methyl]- [2-(5,5-dimethyl - [1,3,2]-dioxoborinan-2-yl)-benzyl]-methylamine 6

Silylether amine **5** (2.2 gm, 6.03 mmol), potassium carbonate (K₂CO₃) (0.9 gm, 8.43 mmol) and 2-(2-bromomethyl-phenyl)-5,5-dimethyl-[1,3,2]-dioxaborinane (2.3 gm, 8.43 mmol) were dissolved in dry MeCN (100 mL) after which the reaction mixture was heated under reflux for 25 h under a N₂ atmosphere. After completion (monitored by TLC), the insoluble suspension was removed by filtration and the filtrate evaporated to give a yellow oil that solidified when treated with ethyl acetate. After recrystallisation from CH₂Cl₂:ethyl acetate (1:1, v/v), amine boronate **6** was obtained (1.6 gm, 47%) as a yellow solid. HRMS: m/z calc. for C₃₅H₄₆NO₃Si: 568.34128 [M+H]⁺; found: 568.34210 [M+H]⁺.

### 9-Hydroxymethyl-10-[N-methyl-N-(o-boronobenzyl)amino]methyl-anthracene 7

Amine boronate **6** (1.5 gm, 2.64 mmol) was dissolved in dry THF (60 mL), with added TBAF (2.0 gm, 7.92 mmol, and the reaction mixture was stirred at room temperature for 25 h under a N₂ atmosphere. After completion (monitored by TLC), the organic solvent was removed under reduced pressure, and residue dissolved in CH₂Cl₂ (150 mL), then washed with water (200 mL). Following this, the organic layer was dried over anhydrous sodium sulfate, concentrated, then purified by column chromatography, on neutral alumina oxide (~150 mesh size), eluting with CH₂Cl₂:MeOH (100:0.5, *v*/*v*) to yield deprotected amine boronate **7** (0.861 gm, 84%) as a pale yellow solid product. Mp: 218-219 °C. IR(ATR): 3436, 3054, 2949, 2849, 2793, 1596, 1443, 1334, 1207, 1118, 1083, 996, 865, 804, 744 cm⁻¹. ¹H NMR (DMSO-d₆, 400 MHz): δ 8.56-8.51 (4H, m, Ar-*H*), 7.60-7.58 (4H, m, Ar-*H*), 7.07-7.02 (2H, m, Ar-*H*), 6.70 (1H, t, *J =* 5.6 Hz, *J* = 7.6 Hz, Ar-*H*), 5.46 (2H, d, *J* = 6.4 Hz, Ar-*H*), 5.46 (2H, s, Ar-C*H*₂-OH), 5.34 (1H, s, ArCH₂-*OH*), 4.64 (2H, s, Ar-C*H*₂-N-), 3.77 (2H, s, Ar-C*H*₂-N-), 2.19 (3 H, s, -N-C*H*₃). ¹³C NMR (DMSO-d₆, 100.26 MHz): δ 156.48, 133.77, 130.55, 129.67, 129.51, 129.23, 128.11, 125.61, 125.46, 125.28, 125.00, 122.88, 118.72, 115.10, 57.57, 55.53, 52.39, 40.86. HRMS: m/z calc. for C₂₄H₂₄BNO₃: 386.19275 [M+H]⁺; found: 386.19214 [M+H]⁺.

### 10-[N-methyl-N-(o-boronobenzyl)aminomethyl]-anthracene-9-methyl methacrylate (ABAM)

Deprotected amine boronate **7** (0.7 gm, 1.81 mmol), methacrylate anhydride (0.840 gm, 5.45 mmol), and DMAP (0.665 gm, 5.45 mmol) were dissolved in dry CH₂Cl₂ (60 mL), then the resulting reaction mixture was stirred at room temperature for 48 h under a N₂ atmosphere. After completion (monitored by TLC), the reaction mixture was washed with 5% w/v aqueous sodium bicarbonate (NaHCO₃) (100 mL), water (100 mL), and lastly with brine solution (100 mL). Following this, the organic layer was dried over anhydrous sodium sulfate, concentrated, then purified by column chromatography, on neutral alumina oxide (~150 mesh size), eluting with CH₂Cl₂:MeOH (100:0.5, v/v) to yield ABAM (0.561 gm, 68%) as a yellow solid product. Mp: 119-121 °C. IR(ATR): 3647, 3054, 2950, 2925, 2849, 2780, 1712, 1481, 1445, 1311, 1209, 1184, 1145, 1006, 940, 812, 746 cm⁻¹. ¹H NMR (CD₃OD, 400 MHz): δ 8.59-8.49 (3H, m, Ar-*H*), 8.10 (2H, s, Ar-B-(O*H)₂*), 7.82 (1H, s, Ar-*H*), 7.70-7.63 (6H, m, Ar-*H*), 7.58-7.56 (2H, m, Ar-*H*), 6.26 (2H, s, Ar-C*H*₂-O-), 5.98 (1H, s, -CO-C-(CH₃)-CH-*H*), 5.56 (1H, s, -CO-C-(CH₃)-C*H*-H), 5.39 (2H, s, Ar-C*H*₂-N-), 4.71 (2H, s, Ar-C*H*₂-N-), 2.76 (3H, s, -N-C*H*₃), 1.87 (3H, s, -CO-C-*(CH₃)*-CH₂). ¹³C NMR (CD₃OD, 100.26 MHz): δ 168.41, 137.45, 135.60, 133.84, 132.37, 132.24, 131.83, 131.51, 130.57, 128.71, 127.77, 127.68, 126.64, 126.52, 124.74, 124.20, 73.02, 63.24, 60.32, 51.60, 41.35, 18.35. HRMS: m/z calc. for C₂₈H₂₈BNO₄: 453.21114 [M]⁺, 454.21897 [M+H]⁺; found: 453.21059 [M]⁺, 454.21827 [M+H]⁺. Previously reported data ESI-MS (MeOH) *m*/*z*: 482.2 [M, -2H₂O, +2CH₃OH, + H]⁺.

### Example 2: Characterization of fluorescent properties of ABAM

A steady-state fluorescence spectroscopy of ABAM was performed, demonstrating an excitation band at 370-390 nm and an emission band at 410-520 nm (Figure 2A). An excitation-emission map (EEM) was also acquired (Figure 2B). The excitation-emission maps (EEMs) were recorded by multifunctional microplate reader Synergy H1 Hybrid Reader (BioTek, USA). The prepared sample solutions of ABAM in 30 mM PBS buffer pH 7.4 (25 µM) were pipetted (100 µL) into a UV-transparent CoStar flat-bottom 96 well microtiter plate (Corning, USA). For both excitation and emission measurements, the slit width was set to 2 nm and gain 100. The excitation and emission ranges during each measurement were set to 250-530 and 300-700 nm (wavelength interval of 10 nm), respectively.

### Example 3: Optimization of assay conditions

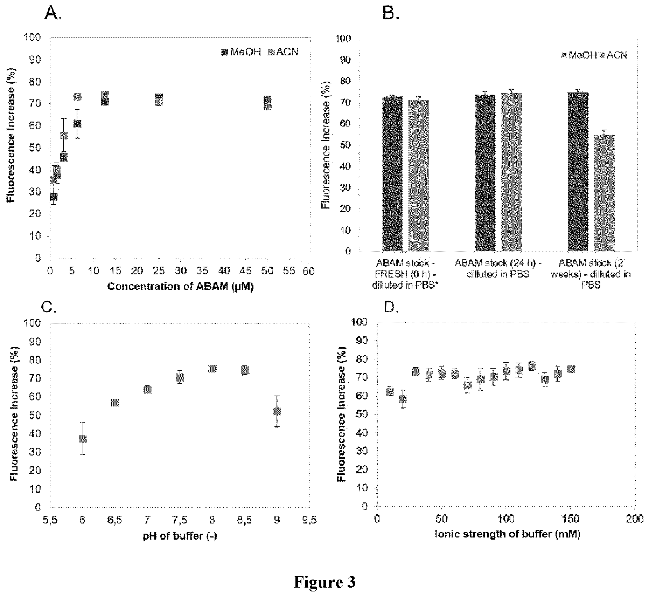

Relative impacts were investigated of polar protic solvent MeOH on fluorescence intensity increases following ABAM/D-fructose (D-Fru) interactions. ABAM taken from a stock solution (>15 µM) in MeOH gives optimal fluorescence intensity increases. Fluorescence intensity increases from ABAM/D-Fru interactions were found further optimal in aqueous conditions comprising 30 mM PBS, pH 7.4 (Figure 3). ABAM was typically pre-dissolved in MeOH, to give an aggregation free primary stock solution, then diluted for use (typically to 25 µM for polyol interaction studies) in 30 mM PBS, pH 7.4.

The impact of pH on fluorescence intensity increases resulting from ABAM/polyol interactions is in keeping with the most recent "loose bolt" internal conversion mechanism, which is nowadays largely favored over the N-B bond theory. According to this "loose bolt" internal conversion mechanism, below a pH defined by pKa₁, PET-mediated suppression of anthracene fluorescence is prevented by amine functional group protonation with or without polyol interactions. Thereafter, in a pH range between pKa₁ and pKa₂, solvent insertion in the absence of polyol interactions results in the formation of saturated anionic boronate species (R-B(OH)₃⁻ ) that can act on their own to take up excited electronic state energy and thus quench anthracene fluorescence. This quenching effect is eliminated with boronate ester formation following polyol addition, for example following the addition of D-Fru. Finally, at pH values above pKa₂, PET-mediated suppression of anthracene fluorescence becomes fully enabled with or without polyol interactions by amine functional group deprotonation. It has been observed that said "loose bolt" internal conversion mechanism is largely uninfluenced by ionic strength.

In the study of the solvent effect, the stock solution of ABAM was prepared in two different solvents: 1) in MeOH and 2) in MeCN to give stock solutions of ABAM (final concentration 2 g/L). From each stock solution, a fluorescence calibration series was prepared by diluting ABAM in 30 mM PBS buffer, pH 7.4, over the concentration range 50-0.2 µM. Aliquots (100 µL) from each concentration were then pipetted into a CoStar flat-bottom 96 well microtiter plate (Corning, USA). Subsequently, D-Fru (1.8 µL, 111 mM) was dissolved (final concentration 2 mM) in 30 mM PBS, pH 7.4, then added to ABAM solution aliquots. Each mixture was analyzed by fluorescence spectroscopy after 10 min of reaction using a Synergy H1 Hybrid Reader (BioTek, USA) with excitation at 370 nm and emission over the range 410-520 nm. Experiments were performed in triplicate, and the results are presented as mean ± standard deviation. In the case of pH and ionic strength investigations, the protocol differed slightly. D-Fru (dissolved in ACS reagent water) was added to ABAM (25 µM) prepared from dilution of MeOH stock solution into 20-150 mM PBS buffer, pH 6-9. The rest of the protocol was the same as above. A technical summary of the assay is presented in Table 3. The optimal assay conditions determined are summarised in Table 3 as well.

**Table 3: Technical features for ABAM-based assay. See Table 2 for buffer abbreviations**

| **Technical features** | |
|---|---|
| Buffers compatible with ABAM -based assay | Buffers which have buffering range around pH 7.4 such as PBS, HEPES, Tris, MOPS, PIPES, TES, BES, Trizma base, etc. |
| pH of appropriate buffer | 6.5 - 8.5 |
| Ionic strength of appropriate buffer | 20 - 150 mM |
| Range of sample (plasma) dilution | 30 - 50x |
| Organic solvent appropriate for storage of ABAM | MeOH |
| Suitable concentration of stock solution of ABAM in MeOH | 2 - 5 g/L |
| Resulting ABAM concentration for determination of glycated proteins in plasma | 5 - 10 µM |
| Interaction time between glycated proteins and ABAM | 10 - 30 min |
| Excitation of ABAM | 370 - 390 nm |
| Fluorescence emission of ABAM | 410 - 520 nm |

### Example 4: Determination of gHSA levels in human plasma

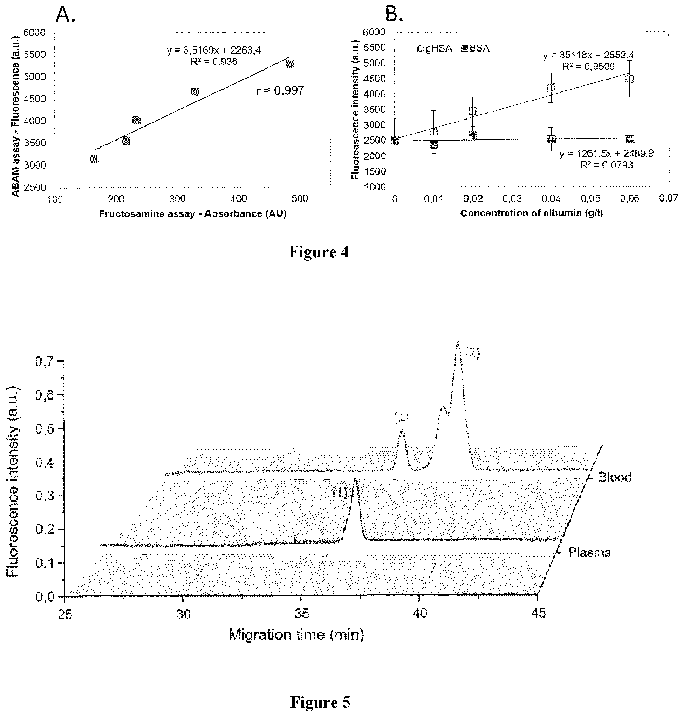

A comparison between the ABAM-based and the fructosamine assays for the determination of gHSA levels in human plasma was performed. The fructosamine assay (ab228558, Abcam, Cambridge, UK) was used according to manufacturers' instructions. Samples of human plasma (purchased from Zen-Bio Inc., USA) were first 50-times diluted with pure ACS reagent water then spiked with sequential additions of gHSA (0.01 to 0.06 g/L) so that total gHSA concentrations fell within the gHSA concentration range diagnostic for both prediabetes and then diabetes. Both assays were found to detect successfully the increased concentrations of gHSA in plasma after spiking. Moreover, both methods showed very similar lower limits of detection (LODs) of gHSA above the plasma baseline concentration (ABAM-based assay - 5.04 mg/L and Fructosamine assay - 5.12 mg/L), and upper limits of quantification (LOQs) of gHSA above the plasma baseline concentration (ABAM-based assay - 65.28 mg/L and Fructosamine assay - 65.53 mg/L). Finally, the correlation between ABAM-based assay and the fructosamine assay was examined by Pearson correlation analysis. The coefficient of determination of 0.997 indicated a significant positive correlation between the two analytical assay methods (see Figure 4A).

Studies were then performed to compare gHSA detection with a non-glycated BSA control. As above, human plasma was 50-times diluted with pure ACS reagent water, then spiked with various concentrations of gHSA or bovine serum albumin (BSA) (negative control) (both from Sigma-Aldrich, St. Louis, MO, USA) in the concentration range from 0.01 to 0.06 g/L (specifically 0.01; 0.02; 0.04 and 0.06 g/L), corresponding to added protein concentrations post spiking of 0.15, 0.3, 0.6 and 1.0 µM, respectively, above the plasma baseline concentration of gHSA. Diluted human plasma (50 µL) or spiked plasma samples (50 µL), as appropriate, were pipetted into CoStar flat-bottom 96-well microtiter plate (Corning, USA). Subsequently, ABAM stock solution (4.4 mM of ABAM in MeOH) was diluted with ACS reagent water to a concentration of 200 µM and then added to either human plasma samples or spiked human plasma samples (final concentration of ABAM in each sample was 7.5 µM), then after 10 min of reaction each mixture was analysed by fluorescence spectroscopy using a Synergy H1 Hybrid Reader (BioTek, USA) with excitation at 370 nm and emission from 410 to 520 nm.

**Table 4: Determination of gHSA and BSA in plasma using ABAM-based assay**

| **Dilute d plasm a (µL) *** | **Stock solution of protein (µL) * *** | **Distille d water (µL)** | **Final concentratio n of spiked protein in plasma (g/L)** | **ABAM diluted solutio n (µL) ***** | **Fluorescent intensity (a.u.) for gHSA****** | **Fluorescent intensity (a.u.) for BSA****** | **Differenc e between gHSA and BSA** |
|---|---|---|---|---|---|---|---|
| 50 | 0 | 3 | 0 | 2 | 2477 ± 736 | 2518 ± 118 | -41 |
| 50 | 0.5 | 1.5 | 0.01 | 2 | 2760 ± 724 | 2358 ± 255 | 401 |
| 50 | 1 | 2 | 0.02 | 2 | 3430 ± 471 | 2659 ± 324 | 771 |
| 50 | 2 | 1 | 0.04 | 2 | 4190 ± 485 | 2529 ± 388 | 1662 |
| 50 | 3 | 0 | 0.06 | 2 | 4469 ± 587 | 2549 ± 81 | 1920 |

| | | | | | | | |
|---|---|---|---|---|---|---|---|
| * Plasma sample was diluted with ACS reagent (distilled) water 50x ** Stock solution of gHSA or BSA (concentration 1g/L) ***ABAM diluted with ACS reagent water (concentration 200 µM) - prepared by mixing of 4.5 µL of stock ABAM solution with 95.5 µL ACS reagent water. Stock solution of ABAM in MeOH (concentration 4.4 mM). * * * *The fluorescence intensities used were subtracted for excitation at a wavelength of 426 nm | | | | | | | |

ABAM-based assays were performed in triplicate, and the results are presented as mean ± standard deviation. Fluorescence data are displayed (Figure 4B). The exact additions of individual components and the measured results are also shown (Table 4). From statistical evaluations, when fluorescent data measured using plasma samples from healthy, prediabetic and diabetic subjects, are converted into concentration data using the assay calibration curve, a given subject is said to be prediabetic when their total plasma concentration of gHSA is in the range 70 to 100 µM, and diabetic when their total gHSA plasma concentration is higher than 100 µM.

Thereafter, given that the fructosamine assay is known to be influenced by various interferents in solution (e.g. vitamin C, bilirubin, urea, high protein concentrations, etc.), the ABAM-based assay was also studied in the presence of several key potential interferents such as saccharides and vitamin C (ascorbic acid). Monosaccharides inevitably bind to ABAM and might be major interferents, in particular the monosaccharides D-Fru, D-glucose (D-Glc), and D-mannose (D-Man) that are all commonly present in human plasma or blood. Therefore, these were studied alongside sorbitol which is created during the metabolic interconversion of D-Glc to D-Fru. In addition, interactions with monosaccharides present on the surface of red blood cells were also studied, specifically galactose (D-Gal), N-acetyl-D-glucosamine (D-GlcNAc) and sialic acid (D-NeuNAc).

In order to perform these interference studies, aliquots of monosaccharide (D-Fru, D-Glc, D-Man, D-Gal, D-NeuNAc, Sorbitol or D-GlcNAc) or ascorbic acid solution (100 µL) were dispersed into 30 mM PBS buffer, pH 7.4. Samples of potential interferents were then prepared by two-fold serial dilutions over a concentration range of 20-0.01 g/L and then pipetted into a CoStar flat-bottom 96-well microtiter plate (Corning, USA). Subsequently, ABAM stock solution in MeOH (4.4 mM) was added (0.57 µL, giving a final ABAM concentration of 25 µM), and after 10 min the fluorescence was determined using a Synergy H1 Hybrid Reader (BioTek, USA) with excitation at 370 nm and emission over the range 410-520 nm. The fluorescence intensities were subtracted for excitation at a wavelength of 426 nm. Experiments were performed in triplicate, and the lower concentration results are presented as mean ± standard deviation (Table 5). These data demonstrate that each of these potential interferents, at their correct physiological concentrations, is essentially undetectable by ABAM-based assay in plasma after 50-fold dilution. D-Glc alone is on the detection limit, but is rendered undetectable when the ABAM-based assay was performed using the much lower ABAM concentration of 7.5 µM, as used for gHSA detection.

**Table 5: Studies of fluorescence effects with different potential interferents. A Summary of interferent molecular weight and concentration data**

| **Potential interferent*** | **Molecular weight (g/mol)** | **Physiological concentration (mol/L)** | **Physiological concentration (g/L)** | **Assay concentration (50x diluted) (g/L)** |
|---|---|---|---|---|
| D-GlcNAc | 221 | 1·10⁻⁷ | 221·10⁻⁵ | 4.42·10⁻⁷ |
| D-NeuNAc | 309 | 1.9·10⁻³ | 5.87·10⁻¹ | 1.17·10⁻² |
| Sorbitol | 182 | 3.9·10⁻⁶ | 7.09·10⁻⁴ | 1.42·10⁻⁵ |
| D-Fru | 180 | 8·10⁻⁶ | 1.44·10⁻³ | 2.88·10⁻⁵ |
| D-Glc | 180 | 5.5·10⁻³ | 9.9·10⁻¹ | 1.98·10⁻² |
| D-Man | 180 | 4.1·10⁻⁵ | 7.38·10⁻³ | 1.48.10-⁴ |
| D-Gal | 180 | 1.5·10⁻⁶ | 2.7·10⁻⁴ | 5.4·10⁻⁶ |
| Vitamin C | 176 | 8·10⁻⁵ | 1.41·10⁻² | 2.82·10⁻⁴ |

| | | | | |
|---|---|---|---|---|
| * abbreviation: D-GlcNAc: N-acetyl-D-glucosamine; D-NeuNAc: sialic acid; D-Fru: D-fructose; D-Glc: D-glucose; D-Man: D-mannose; D-Gal: D-galactose; Vitamin C: ascorbic acid | | | | |

B Summary of concentration dependent interference responses using ABAM-based assay. Responses for each potential interferent at their assay concentration are shown shaded

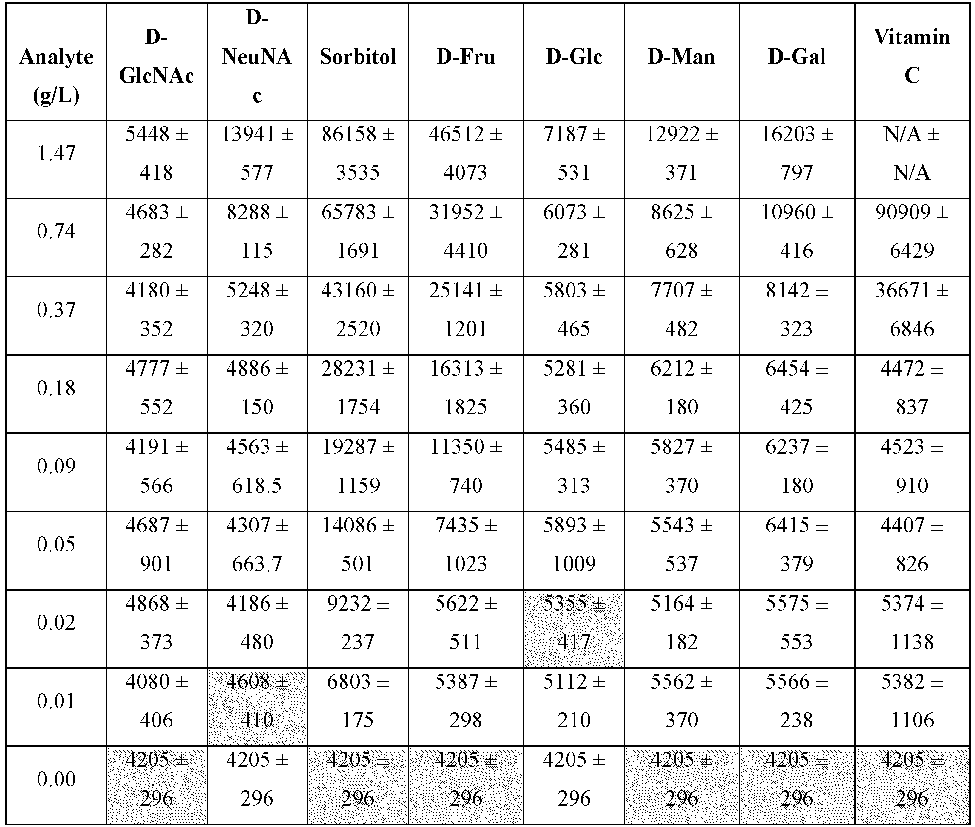

In comparison with the commercially available fructosamine assay, our newly developed ABAM-based assay has several advantages (Table 6). In particular, the assay is selective in plasma for the detection of glycated protein gHSA, whilst the fructosamine assay is more nonspecific. The analysis time of our ABAM-based assay is **twelve times shorter,** and the overall procedure is **more user-friendly** because it works only with only one reagent, and does not require heating to a specific temperature as in the case of fructosamine assay. Another key advantage is that detection of glycated proteins with our ABAM-based assay is unlikely to be **compromised by molecular interference** from known potential interferents. Moreover, our ABAM-based assay is more than **six times less expensive** to operate and the **amount of sample** necessary is **ten times lower.** All these advantages make our ABAM-based assay a promising competitor to currently available methods for the determination of pathogenic glycated protein levels in human blood samples.

**Table 6: Comparison of the newly developed ABAM-based assay with the commercially available fructosamine assay.**

| **Parameter** | **ABAM-based assay** | **Fructosamine assay** |
|---|---|---|
| Plasma sample volume | 1 µL | 10 µL |
| Calculated LOD above the gHSA plasma baseline | 5.04 mg/L of gHSA | 5.12 mg/L of gHSA |
| Calculated LOQ above the gHSA plasma baseline | 65.28 mg/L of gHSA | 65.53 mg/L of gHSA |
| Time of assay | 5 min | 60 min |
| Price | 100 € / 100 tests | 660 € / 100 tests |
| Interference | N/A | Temperature, vitamin C, bilirubin, urea, and total protein levels |
| Plasma Specificity | gHSA | All glycated plasma proteins |

Example 5: *CE-LIF analysis of blood and plasma samples using ABAM fluorescence detection.* Plasma and blood samples were mixed with ABAM as above, then capillary electrophoresis (CE) experiments were performed on an Agilent 7100 System (Agilent technologies, Waldbronn, Germany) equipped with laser-induced fluorescence (LIF) detector (ZetaLIF, Picometrics, Toulouse, France) with solid-state laser (λₑₘ, 360 nm) as an excitation source. A fused silica capillary was used with an internal diameter of 75 µm, total length of 64.5 cm and effective length of 56 cm. The capillary was flushed with 1 M NaOH for 10 mins and for 10 mins with background electrolyte (BGE) prior to first use. The capillary was also rinsed for 60 s with 1 M NaOH and for 180 s with BGE before each run to obtain stable measurements.

Samples were injected hydrodynamically with an applied pressure of 10 mBar for 3 s. Separation voltage was set to 10 kV. The capillary cassette temperature was kept at 25 °C throughout, while the background electrolyte composition was 30 mM borate buffer with 20 mM sodium dodecyl sulphate and ethanol 7% (v/v). Electrolyte solutions were filtered with 0.25 µm polyvinylidene fluoride (PVDF) syringe filters (Sigma-Aldrich, St. Louis, MO, USA).

Using the capillary electrophoresis system equipped with a laser-induced fluorescence detector (CE-LIF), electropherograms were obtained from analyses of whole blood and corresponding plasma samples, following application of our ABAM-based method (as above) to label proteins. Data showed (Figure 5), that glycated hemoglobin and gHSA can be detected in blood samples. This correlates with expectations given that blood contains glycated hemoglobin within erythrocytes and gHSA in plasma. Once corresponding plasma samples were prepared by removal of blood erythrocytes, then only gHSA was detected and no other glycated proteins, even though gHSA actually only constitutes 44% of the total glycated proteins in plasma. These data demonstrate conclusively that ABAM interacts preferentially with gHSA in preference to all other glycated proteins in plasma. Therefore, our ABAM-based assay does indeed preferentially and primarily detect gHSA in plasma samples.

### Conclusion:

Overall, the correlation between our ABAM-based assay and the fructosamine assay was examined by Pearson correlation analysis. The coefficient of determination of 0.997 indicated a significant positive correlation between the two analytical methods studied (see Figure 3A). In addition, the ABAM assay is able to measure gHSA level in the dynamic range which correlates with the conditions of prediabetes to diabetes. Furthermore, the fructosamine assay is known to be influenced by various analytes in solution (e.g. vitamin C, bilirubin, urea, high protein concentration, etc.). When, our ABAM-based assay was also studied in the presence of several key analytes such as Vitamin C, monosaccharides, and non-glycated proteins (represented by different concentrations of BSA), no obvious impact on the output of the ABAM-based assay was observed. Accordingly, we can rule out the possibility that ABAM/ascorbic acid, ABAM/monosaccharide and ABAM/non-glycated protein interactions might potentially interfere with the accurate determination of glycated protein levels using our ABAM-based assay.

## Claims

1. A method of detection of glycated proteins in biological samples **characterized in that** it comprises the following steps:
i) providing 10-[*N*-methyl-*N*-(o-boronobenzyl)aminomethyl]-anthracene-9-methyl methacrylate (ABAM);
ii) preparing a solution of ABAM in a biocompatible buffer with pH in the range of from 6.5 to 8.5 or in *in vitro* blood plasma of a healthy subject;
iii) measuring initial fluorescence intensity of ABAM solution from step ii) at the wavelength in the range of from 410 to 520 nm;
iv) *in vitro* mixing of a known amount of ABAM with a known amount of a biological sample, so that the final concentration of ABAM is the same as in step ii);
v) measuring fluorescence intensity of the mixture from step iv) at the same wavelength as in step iii);
vi) determining the fluorescence intensity increase between the initial fluorescence intensity measured in step iii) and the fluorescence intensity of the mixture of ABAM and biological sample measured in step v);
vii) determining the presence and optionally the amount of glycated proteins in the biological sample.

2. The method according to claim 1, **characterized in that** the biological sample is selected from the group comprising blood, plasma, urine, tissue, cells and saliva, preferably the biological sample is plasma, more preferably human blood plasma.

3. The method according to claim 1 or 2, **characterized in that** step vii) is performed by comparing the fluorescence intensity increase from step vi) with statistical evaluation of the fluorescence intensity increase in biological samples of healthy subjects, prediabetic subjects, and diabetic subjects.

4. The method according to claim 2 or 3, **characterized in that** step vii) is performed by determining the amount of gHSA in the human plasma sample by plotting the fluorescence intensity increase from step vi) into a calibration curve;
wherein the calibration curve is prepared using at least three calibration plasma samples from healthy subject enriched with added various known additional quantities of gHSA in a concentration range of from 0.01 to 0.06 g/L;
fluorescence intensities of the calibration plasma samples are measured according to the steps i) to vi) of claim 1;
the difference in fluorescence intensity of each calibration sample compared to the fluorescence intensity of ABAM solution from step iii) is then plotted as a function of the added gHSA concentrations.

5. The method according to any one of the preceding claims, **characterized in that** the ABAM solution in step ii) has concentration in the range of from 5 to 50 µM, preferably from 5 to 25 µM.

6. The method according to any one of the preceding claims, **characterized in that** the biocompatible buffer in step ii) is selected from the group comprising PBS, HEPES, Tris, MOPS, PIPES, Trizma base, TES, BES.

7. The method according to any one of the preceding claims, **characterized in that** the ABAM concentration of the final mixture in step iv) is in the range of from 5 to 10 µM, preferably 7.5 µM.

8. The method according to any one of the preceding claims **characterized in that** fluorescence emission intensities in step iii) and step v) are measured with an excitation wavelength in the range of from 370 to 390 nm and emission wavelength in the range of from 410 to 520 nm.

9. The method according to any one of the preceding claims **characterized in that** the fluorescence intensity in step v) is measured after at least 10 mins from mixing ABAM with the biological sample, preferably, the fluorescence intensity in step v) is measured in the time range of from 10 mins to 30 mins after mixing ABAM with the biological sample.

10. Use of the method according to any one of the preceding claims to determine concentrations of gHSA in plasma samples.

11. Use of the method according to any one of the preceding claims 1 to 9 in medicine *in vitro,* preferably for *in vitro* diagnostics of diabetes mellitus.
